# EUROPEAN PATENT APPLICATION

(11) **EP 4 734 120 A1**
(43) Date of publication of application: **29.04.2026**
(21) Application number: 24208556.1
(22) Date of filing: 24.10.2024
(51) Int. Cl.: G16H 10/40

(54) **METHOD OF SAMPLE IDENTIFICATION, SAMPLE PROCESSING SYSTEM, AND NON-TRANSITORY STORAGE MEDIUM**

(71) Applicant: Roche Diagnostics GmbH, 68305 Mannheim (DE); Roche Diagnostics International AG, 6343 Rotkreuz (CH)
(72) Inventor: BRADBURY, Christopher, 6343 Rotkreuz (CH); SCHNEE, Sandro, 68305 Mannheim (DE); HERBEL, Petra, 68305 Mannheim (DE)
(74) Representative: Keinhorst, Volker Gerhard

(57) **Abstract**

The present disclosure concerns a method of sample identification of a sample in a sample processing system, comprising: registering, for each sample of a plurality of samples to be processed by the sample processing system, initial sample identification data uniquely identifying the respective sample among the plurality of samples; registering, for each sample of the plurality of samples, initial sample fingerprint data indicative of an internal state of the respective sample; associating, for each sample of the plurality of samples, the respective initial sample identification data with the respective initial sample fingerprint data; determining that a sample identification condition is met, wherein the sample identification condition is indicative of a need to identify a specific sample being processed by the sample processing system; measuring, for the specific sample, current sample fingerprint data indicative of a current internal state of the specific sample; and mapping the current sample fingerprint data to the initial sample fingerprint data of one sample of the plurality of samples in order to identify the specific sample. The present disclosure further concerns a corresponding sample processing system and non-transitory computer-readable storage medium.

## Description

The present invention concerns a method of sample identification, a sample processing system configured to process samples, and a non-transitory storage medium.

The present invention lies in the field of sample tracking, specifically in the field of sample tracking and identification in sample processing systems.

In conventional sample processing devices, samples are either not tracked or identified at all, or provided with an external tag to allow identification thereof. The inventors realised during the conception of the present invention that such an approach presents multiple drawbacks. On the one hand, the provision of external tags to samples requires additional processing steps. On the other hand, external tags may get damaged, mixed up, or removed, which results in the corresponding sample to no longer be identifiable.

It is therefore an object of the present invention to provide means of sample identification and/or tracking having improved operational characteristics, such as for example improved reliability, precision, and sample tracking.

The above object is solved by a method of sample identification, a sample processing system configured to process samples, and a non-transitory storage medium having the features recited in the independent claims, respectively. Preferred embodiments form the subject of the dependent claims.

A first aspect of the present disclosure concerns a method of sample identification of a sample in a sample processing system. The sample processing system may be configured to process at least one sample, preferably a plurality of samples.

In other words, the method may additionally comprise at least a step of processing one or more samples and/or at least a step of transporting one or more samples within the sample processing system. The step of processing one or more samples may in particular occur at least after the step of associating, as described further below. Processing the sample may comprise performing one or more desired analyses on the one or more sample. However, it is understood that processing the one or more samples is not limited thereto, and thus alternative and/or additional processing steps may also be included. For example, processing one or more samples may comprise one or more of preparing the one or more samples, treating the one or more samples, pre-treating the one or more samples, storing the one or more samples, heating the one or more samples, freezing the one or more samples.

The sample processing system may, for example, be an in-vitro diagnostic analyzer. However, other types of sample processing systems may also be used, such as other types of analytical systems and/or analytical devices. The sample processing system may further comprise one or more features of a sample processing system, as described herein and/or shown in the appended Figures.

The method further comprises a step of registering, for each sample of a plurality of samples to be processed by the sample processing system, initial sample identification data uniquely identifying the respective sample among the plurality of samples. The plurality of samples may comprise one or more samples. Uniquely identifying may be understood herein such that a one-to-one relationship may exist between each respective sample and the corresponding initial sample identification data. Therefore, in other words, it may be possible to identify a respective sample among the plurality of samples based on the corresponding initial sample identification data. Furthermore, "registering" data may be understood herein as comprising and/or corresponding to receiving data, measuring data, and/or storing data. The initial sample identification data may, for example, be stored in an initial sample identification database.

The method further comprises registering, for each sample of the plurality of samples, initial sample fingerprint data indicative of an internal state of the respective sample. The internal state of the respective sample may be a physical and/or chemical state of the respective sample. Furthermore, it may be understood that the internal state of the respective sample may be substantially unique to said respective sample. Thus, for example, any two substantially different samples may consequently have substantially different respective internal states. The initial sample fingerprint data may, for example, be stored in an initial sample fingerprint database.

Furthermore, the method may further comprise determining whether the initial sample fingerprint data of two samples of the plurality of samples is substantially identical. The initial sample fingerprint data of two samples may be understood to be substantially identical if a similarity value between the respective initial sample fingerprint data exceeds a corresponding similarity threshold. The similarity value between two initial sample fingerprint data may be computed in a conventional way (i.e., as a similarity between two datasets), by choice of the user and/or based on the operational requirements of the method. For example, similarity may be defined as the total area between two initial sample fingerprint data (e.g., between two spectral datasets). The similarity threshold may be predetermined and/or input by the user and/or determined based on the operational requirements of the method and/or of the sample processing system. If it is determined that the initial sample fingerprint data of two samples of the plurality of samples is substantially identical, a corresponding output may be presented, for example as a corresponding alert to a user, and/or further processing of the respective two samples by the sample processing system may be (at least temporarily) halted and/or prevented. Alternatively, one of the two samples may be further processed, while processing of the second of the two samples may be halted and/or prevented. Thereby it may be efficiently prevented that the sample processing system processes two samples having substantially identical initial sample fingerprint data at the same time, thus improving overall reliability.

In particular, it may be possible to register the initial sample fingerprint data without processing the sample by adding additional marker substances thereto. In other words, the internal state of the respective sample may be an internal state of the respective sample without the addition of further marker substances. This allows for a much more efficient fingerprinting of the respective sample while at the same time reducing potential contamination and minimizing necessary handling steps.

The initial sample identification database and the initial sample fingerprint database may be implemented as a common and/or single database. Alternatively, the initial sample identification database and the initial sample fingerprint database may be implemented as separate databases.

The method further comprises associating, for each sample of the plurality of samples, the respective initial sample identification data (of the respective sample) with the respective initial sample fingerprint data (of the respective sample). Said associating may comprise generating, for each sample of the plurality of samples, an association data element, such as a link element and/or reference element, wherein the association data element is configured to associate the initial sample identification data with the initial sample fingerprint data for the respective sample. Each association data element and/or each association may be unique to the respective sample. The plurality of associations and/or the plurality of association data elements and their respective initial sample identification data and initial sample fingerprint data may be stored as an association table. For example, an association element may be a table row and/or column associating the initial sample identification data with the initial sample fingerprint data for the respective sample. The plurality of associations and/or the plurality of association data elements may be stored in a sample fingerprint reference database. Alternatively, the plurality of associations and/or the plurality of association data elements may be stored in the common database, the initial sample identification database, and/or the initial sample fingerprint database.

The method further comprises determining that a sample identification condition is met. The sample identification condition is indicative of a need to identify a specific sample being processed by the sample processing system. The sample identification condition may comprise a non-identification condition indicative of the sample being not conventionally identifiable.

For example, the sample identification condition may comprise an unreadable- and/or missing-label condition indicative of a label of the respective sample not being readable and/or missing from the sample. Such a condition may, for example, occur during processing or handling of the sample (e.g., by damaging or unintentionally removing the label), and would conventionally lead to the sample being discarded. Determining that the unreadable- and/or missing-label condition is met may comprise verifying, for example by an optical sensor, by another type of sensor and/or by a user, the presence and/or state of the label of the sample. If the label is considered to be unreadable and/or missing, the unreadable- and/or missing-label condition may be determined to be met.

For example, the sample identification condition may comprise a lost-sample condition indicative of a sample not being in a predicted and/or desired location, for example within the sample processing system. Such a condition may, for example, occur when a sample collides with another element/entity or is otherwise undesirably displaced during processing. Similarly, such a condition may, for example, occur if the sample processing system comprises several diverging and converging sample processing pathways, along which the sample processing system may transport the one or more samples. Determining that the lost-sample condition is met may comprise determining the location of a sample, for example optically, by a user, and/or another type of location sensor, and verifying whether the respective sample is expected and/or required to be in said location. If the respective sample is considered to not be in a valid and/or correct and/or expected location, the lost-sample condition may be considered to be met.

For example, the sample identification condition may comprise an unknown-sample condition indicative of a sample being unknown to the processing system. This situation may for example arise if a sample is provided to the processing system too early or at an unexpected location. Furthermore, this situation may also arise if, for example, a sample is determined to have a readable but unknown or mislabeled sample label. Conventionally, such a sample would need to be discarded or assessed via operator intervention. Determining whether the unknown-sample condition is met may comprise, for example, inspecting, such as optically inspecting, the respective sample. If the respective sample is determined to be unknown, the unknown-sample condition may be considered to be met.

For example, the sample identification condition may comprise an identification verification condition indicative of a request to identify a particular sample. For example, a user may wish to verify the identity of a particular sample and thus input a corresponding identification request. Once such an identification request has been input, the identification verification condition may be considered to be met.

The determining that the sample identification condition is met may be performed prior to any potential processing step configured to substantially change an internal state of the respective sample. The determining that the sample identification condition is met may be performed by inspecting a sample in the sample processing system by one or more sensors, such as optical, electrical, electronic, haptic, and/or weight sensors.

The method further comprises measuring, for the specific sample, current sample fingerprint data indicative of a current internal state of the specific sample. The current internal state of the specific sample may be an internal state of the specific sample at the time of measurement of the current sample fingerprint data. The measuring of the current sample fingerprint data may occur subsequent to the determining that the sample identification condition is met. The method may further comprise a step of transporting the sample, after determining that the sample identification condition is met, to a sample measurement unit and/or station (e.g., a registration unit and/or a second registration sub-unit) of the sample processing system configured to measure the current sample fingerprint data.

The method further comprises mapping the current sample fingerprint data to the initial sample fingerprint data of one sample of the plurality of samples in order to identify the specific sample. The step of mapping may, for example, also encompass matching and/or comparing the current sample fingerprint data to the initial sample fingerprint data of one sample of the plurality of samples in order to identify the specific sample. The step of mapping may comprise comparing the current sample fingerprint data and the initial sample fingerprint data of each sample of the plurality of samples to determine a best fit initial sample fingerprint data. The step of mapping may then, for example, comprise mapping the current sample fingerprint data to the best fit initial sample fingerprint data. The best fit may, for example, be determined by a conventional data matching/comparison algorithm. For example, a further similarity value between the current sample fingerprint data and the initial sample fingerprint data of each sample of the plurality of samples may be determined, e.g., as discussed for the comparison of two initial sample fingerprint data above. The best fit initial sample fingerprint data may be the initial sample fingerprint data having the highest further similarity value. The best fit initial sample fingerprint data may be the initial sample fingerprint data having the highest further similarity value, wherein the highest further similarity value may exceed a further similarity threshold. Setting the further similarity threshold may in particular serve to prevent unsimilar data being considered a best fit. The further similarity threshold may be set according as an operator choice, and may be determined based on the sample processing system and, e.g., the expected variance in processed samples.

In particular, subsequent to mapping the current sample fingerprint data to the initial sample fingerprint data of one sample of the plurality of samples, the method may comprise retrieving and/or determining the initial sample identification data associated with the respective, mapped initial sample fingerprint data to identify the specific sample among the plurality of samples.

The step of mapping the current sample fingerprint data to the initial sample fingerprint data of one sample of the plurality of samples may further comprise determining that the current sample fingerprint data cannot be mapped to the initial sample fingerprint data of one sample of the plurality of samples. Such a situation may, for example, occur when an unknown sample is provided to the sample processing system. The method may then further include outputting a corresponding alert to a user and/or preventing further processing of the specific sample. The method may subsequently include registering, from the user, initial sample identification data for the specific sample and registering the current sample fingerprint data of the specific sample as the initial sample fingerprint data of the specific sample.

In particular, the step of measuring, for the specific sample, current sample fingerprint data indicative of a current internal state of the specific sample, and the step of mapping the current sample fingerprint data to the initial sample fingerprint data of one sample of the plurality of samples in order to identify the specific sample may be performed in response to and/or based on determining that the sample identification condition is met. In other words, the method may further comprise, in response to and/or based on determining that the sample identification condition is met: measuring, for the specific sample, current sample fingerprint data indicative of a current internal state of the specific sample; and mapping the current sample fingerprint data to the initial sample fingerprint data of one sample of the plurality of samples in order to identify the specific sample.

Conventionally, samples processed in a processing system are identified and/or tracked (if at all) by placing an identification tag on the respective sample, such as an external label. Therefore, conventional sample identification approaches rely on the addition of further elements to the respective samples. This not only requires additional processing steps to add said further elements, but also is fully reliant on said further elements continuing to mark said samples. Thus, for example, a broken or unreadable label will prevent the sample from being identified and/or tracked.

In contrast, using the inventive approach of the present disclosure, sample tracking and/or identification is achieved by use of sample fingerprint data indicative of an internal state of the sample. Therefore, it becomes possible to track and/or identify the respective sample at any point in the sample processing system, irrespective of any particular state of external labelling of the sample. In other words, even samples with unreadable or lost external labelling may be tracked and/or identified. This is of particular importance for diagnostic samples.

Consequently, the present invention allows for a reduction in sample loss, improvement in sample tracking and/or identification, reduction and/or prevention in wrong/faulty diagnosis, a reduction and/or prevention in sample contamination and handling, and improved safety.

Furthermore, the method may comprise a step of evaluating the initial sample fingerprint data and/or the current sample fingerprint data and accordingly grouping the plurality of samples into one or more sample groups. For example, the step of evaluating may comprise determining similarities in the initial sample fingerprint data and/or the current sample fingerprint data indicative of the respective samples being drawn from the same individual. Therefore, for example, two or more samples may be grouped into the same group indicating that said two or more samples are drawn from the same user. For example, the steps of evaluating the initial sample fingerprint data and/or the current sample fingerprint data and grouping the plurality of samples into one or more sample groups may be performed using a grouping machine learning model, such as a trained grouping machine learning model. The trained grouping machine learning model may be trained on grouping test data, wherein the grouping test data may comprise a plurality of grouping test data elements. Each grouping test data element may comprise initial sample fingerprint data and/or current sample fingerprint data and a corresponding group label.

Registering the initial sample fingerprint data for a sample may comprise measuring the initial sample fingerprint data for the sample using a measurement unit (e.g., using a registration unit and/or a second registration sub-unit and/or a sample measurement unit of the sample processing system) using a fingerprint measurement calibration setting of the measurement unit. The fingerprint measurement calibration setting may be indicative of and/or comprise measurement parameters of the measurement unit under which the initial sample fingerprint data is measured. Measuring the current sample fingerprint data of a sample may comprise measuring the current sample fingerprint data of a sample using a measurement unit (e.g., using a registration unit and/or a second registration sub-unit and/or a sample measurement unit of the sample processing system), for example using the fingerprint measurement calibration setting of the measurement unit. Thereby, operation of the method, such as the step of mapping, may be significantly improved and facilitated. Furthermore, hardware-based and/or calibration-based deviations can be limited and/or reduced and/or avoided.

The initial sample identification data may comprise a sample ID. The sample ID may be an identification index or number uniquely assigned to and/or determined for a respective sample. In particular, any two samples to be processed by the sample processing system may have different sample IDs. Preferentially, the initial sample identification data for each sample of the plurality of samples may comprise at least the respective sample ID.

The initial sample identification data may comprise sample origin data. The sample origin data may comprise source data indicating a source of the respective sample, for example source data identifying an individual from whom the sample was drawn. The sample origin data may comprise temporal data indicating a date and/or a time of day when the sample was obtained, e.g., drawn from an individual. The sample origin data may further comprise situational data indicating contextual circumstances under which the sample was obtained, wherein the situational data may for example comprise a location of where the sample was drawn, how the sample was drawn, who drew the sample (e.g., a clinic, house doctor, nurse, etc.), and/or a dietary state of the individual from whom the sample was drawn (e.g., on an empty stomach, specific current diet plan, etc.).

The initial sample identification data may comprise a sample processing plan. The sample processing plan may be indicative of processing to be done on the sample during processing of the respective sample by the sample processing system. The sample processing plan may for example comprise a listing of one or more processing steps to be followed for the respective sample to be processed. The sample processing plan may comprise a processing schedule indicative of a sequence of processing steps to be followed for the respective sample to be processed. The sample processing plan may further comprise at least one processing constraint for the sample, such as a maximum amount of time the sample may remain uncooled, an expiry date of the sample, and/or a priority indication of the sample indicating a relative priority of the sample relative to other samples.

The initial sample identification data may comprise sample status data. The sample status data may be indicative of a current status of the respective sample during registration of the initial sample identification data. For example, the sample status data may comprise environmental data, such as for example a current temperature of the respective sample and/or a refrigeration state of the respective sample. The sample status data may comprise physical parameters of the sample, such as a volume of the sample, a composition type of the respective sample (e.g., full blood, plasma, urine, etc.), and/or a physical state of the sample (e.g., solid, liquid, gaseous, in powder form, etc.). The sample status data may further comprise a treatment history of the respective sample indicating at least one treatment step performed on the respective sample between obtaining the sample and the registration of the initial sample identification data (e.g., indicating whether and/or how the sample was previously one or more of centrifuged, diluted, heated, cooled, etc.).

It is understood that the above list of possible initial sample identification data is not to be interpreted as limiting. In particular, other data may be, additionally and/or alternatively, included into the initial sample identification data.

Therefore, by providing such initial sample identification data, a unique correspondence between the respective sample with its respective initial sample identification data may be established. This in turn may further enhance the reliability and precision of the method of sample identification.

Registering the initial sample identification data may comprise receiving the initial sample identification data as input, such as for example by direct operator input and/or from a database. In other words, the initial sample identification data may be provided as a predetermined data packet in a predetermined data format. Alternatively, the method may further comprise a step of measuring and/or determining the initial sample identification data prior and/or concurrent to its registration.

Registering the initial sample fingerprint data may comprise receiving the initial sample fingerprint data as input, such as for example by direct operator input and/or from a database. In other words, the initial sample fingerprint data may be provided as a predetermined data packet in a predetermined data format. This may allow a very efficient registration of initial sample fingerprint data. Alternatively, the method may further comprise a step of measuring and/or determining the initial sample fingerprint data prior and/or concurrent to its registration, e.g., by measurement using a measurement unit. This may result in further improvements in data reliability and precision.

Each sample of the plurality of samples may be one or more of a full blood sample, a blood serum sample, a blood plasma sample, a saliva sample, a urine sample, and a spinal fluid sample. However, it is understood that the present disclosure is not limited to such a list of exemplary sample types, and other forms and/or types of (e.g., biological) sample could also be implemented.

Each sample of the plurality of samples may be one of a solid sample, a liquid sample, or a gaseous sample. A solid sample may be understood to further encompass dried, deposited, and/or frozen samples. A liquid sample may be understood to further also encompass molten and/or condensed samples. A gaseous sample may be understood to further encompass evaporated and/or sublimated samples.

However, the present disclosure is not limited to the above, and it is further also understood that one or more samples of the plurality of samples may change their state during processing. Thus, one or more samples of the plurality of samples may enter into and/or switch between different matter states during execution of the present method and/or during processing by the sample processing system. Furthermore, one or more samples of the plurality of samples me be in two or more matter states simultaneously. For example, a sample may contain solid, liquid, and/or gaseous matter phases.

The initial sample fingerprint data may comprise initial infrared vibrational spectral data of the respective sample. An example of how to obtain infrared vibrational spectral data from a sample may be given in Huber, M., Kepesidis, K.V., Voronina, L. et al.: "Stability of person-specific blood-based infrared molecular fingerprints opens up prospects for health monitoring", Nat Commun 12, 1511 (2021) (https://doi.org/10.1038/s41467-021-21668-5), which is incorporated by reference in its entirety herein.

Specifically, the initial infrared vibrational spectral data may comprise a continuous infrared vibrational spectrum or comprise a segmented infrared vibrational spectrum. A continuous spectrum may be understood herein as a continuously measured spectrum between a lower (e.g., wavelength) bound and an upper (e.g., wavelength) bound. Respective lower and upper bounds may be chosen in accordance with the respective desired spectral data and/or requirements of the respective sample. Merely as an example, lower and upper bounds may be chosen at the near-infrared spectrum, e.g., from approx. 700nm to approx. 2500nm. Merely as an additional example, lower and upper bounds may be chosen at the infrared spectrum, e.g., from approx. 2500nm to approx. 100000nm. Alternatively, lower and upper bounds may be chosen at the microwave spectrum, e.g., from approx. 0.1mm to approx. 1m. However, other bounds are possible, such as for example between approx. 700nm and 1m. A segmented spectrum may be understood herein as a spectrum containing two or more (preferably non-overlapping and/or disjoint) continuous spectra. The current sample fingerprint data may comprise current infrared vibrational spectral data of the specific sample. Specifically, the current infrared vibrational spectral data may comprise a continuous infrared vibrational spectrum or comprise a segmented infrared vibrational spectrum.

Mapping the current sample fingerprint data to the initial sample fingerprint data may comprise mapping the current infrared vibrational spectral data to the initial infrared vibrational spectral data of one of the plurality of samples in order to identify the specific sample. Such mapping may, for example, be implemented by one or more of a matching algorithm, a comparison algorithm, a best-fit algorithm, and/or a trained machine learning model.

The initial sample fingerprint data may comprise initial microwave spectral data (e.g., initial microwave rotational spectral data) of the respective sample. Specifically, the initial microwave spectral data may comprise a continuous microwave spectrum or comprise a segmented microwave spectrum. The current sample fingerprint data may comprise current microwave spectral data (e.g., current microwave rotational spectral data) of the specific sample. Specifically, the current microwave spectral data may comprise a continuous microwave spectrum or comprise a segmented microwave spectrum.

Mapping the current sample fingerprint data to the initial sample fingerprint data may comprise mapping the current microwave spectral data to the initial microwave spectral data of one of the plurality of samples in order to identify the specific sample. Such mapping may, for example, be implemented by one or more of a matching algorithm, a comparison algorithm, and/or a trained machine learning model. Thereby, a particularly efficient means for sample tracking and/or identification may be achieved. Furthermore, thereby (as well as for other initial sample fingerprint data and current sample fingerprint data, as described herein) it may be possible to obtain said data without requiring opening and/or directly accessing/contacting the respective sample.

The initial sample fingerprint data may comprise initial Raman spectral data of the respective sample. Specifically, the initial Raman spectral data may comprise a continuous Raman spectrum or comprise a segmented Raman spectrum. The current sample fingerprint data may comprise current Raman spectral data of the specific sample. Specifically, the current Raman spectral data may comprise a continuous Raman spectrum or comprise a segmented Raman spectrum.

Mapping the current sample fingerprint data to the initial sample fingerprint data may comprise mapping the current Raman spectral data to the initial Raman spectral data of one of the plurality of samples in order to identify the specific sample. Such mapping may, for example, be implemented by one or more of a matching algorithm, a comparison algorithm, and/or a trained machine learning model. Thereby, a particularly efficient means for sample tracking and/or identification may be achieved.

The initial sample fingerprint data may comprise initial NMR (nuclear magnetic resonance) spectral data of the respective sample. Specifically, the initial NMR spectral data may comprise a continuous NMR spectrum or comprise a segmented NMR spectrum. The current sample fingerprint data may comprise current NMR spectral data of the specific sample. Specifically, the current NMR spectral data may comprise a continuous NMR spectrum or comprise a segmented NMR spectrum.

Mapping the current sample fingerprint data to the initial sample fingerprint data may comprise mapping the current NMR spectral data to the initial NMR spectral data of one of the plurality of samples in order to identify the specific sample. Such mapping may, for example, be implemented by one or more of a matching algorithm, a comparison algorithm, and/or a trained machine learning model. Thereby, a particularly efficient means for sample tracking and/or identification may be achieved.

The initial sample fingerprint data may comprise initial absorption (e.g., ultraviolet-visible, UV VIS) spectral data of the respective sample. Specifically, the initial absorption (e.g., ultraviolet-visible) spectral data may comprise a continuous absorption (e.g., ultraviolet-visible) spectrum or comprise a segmented absorption (e.g., ultraviolet-visible) spectrum. The current sample fingerprint data may comprise current absorption (e.g., ultraviolet-visible) spectral data of the specific sample. Specifically, the current absorption (e.g., ultraviolet-visible) spectral data may comprise a continuous absorption (e.g., ultraviolet-visible) spectrum or comprise a segmented absorption (e.g., ultraviolet-visible) spectrum.

Mapping the current sample fingerprint data to the initial sample fingerprint data may comprise mapping the current absorption (e.g., ultraviolet-visible) spectral data to the initial absorption (e.g., ultraviolet-visible) spectral data of one of the plurality of samples in order to identify the specific sample. Such mapping may, for example, be implemented by one or more of a matching algorithm, a comparison algorithm, and/or a trained machine learning model. Thereby, a particularly efficient means for sample tracking and/or identification may be achieved.

The initial sample fingerprint data may comprise initial mass spectrometry spectral data of the respective sample. Specifically, the initial mass spectrometry spectral data may comprise a continuous mass spectrometry spectrum or comprise a segmented mass spectrometry spectrum. The current sample fingerprint data may comprise current mass spectrometry spectral data of the specific sample. Specifically, the current mass spectrometry spectral data may comprise a continuous mass spectrometry spectrum or comprise a segmented mass spectrometry spectrum.

Mapping the current sample fingerprint data to the initial sample fingerprint data may comprise mapping the current mass spectrometry spectral data to the initial mass spectrometry spectral data of one of the plurality of samples in order to identify the specific sample. Such mapping may, for example, be implemented by one or more of a matching algorithm, a comparison algorithm, and/or a trained machine learning model. Thereby, a particularly efficient means for sample tracking and/or identification may be achieved.

The initial sample fingerprint data may comprise initial values of a plurality of physical parameters of the respective sample. The current sample fingerprint data may comprise current values of the plurality of physical parameters of the specific sample. Mapping the current sample fingerprint data to the initial sample fingerprint data may comprise mapping the current values to the initial values of one of the plurality of samples in order to identify the specific sample. Such mapping may, for example, be implemented by one or more of a matching algorithm, a comparison algorithm, and/or a trained machine learning model. Thereby, a particularly efficient means for sample tracking and/or identification may be achieved.

In particular, the plurality of physical parameters may comprise one or more physical parameters, wherein a choice of the one or more physical parameters may be based on the plurality of samples to be processed.

The one or more physical parameters may comprise one or more of a temperature of the respective sample, a matter state of the respective sample, a volume of the respective sample, a vessel type of the respective sample, a weight of the respective sample, a conductivity of the respective sample, an impedance of the respective sample, and/or a centre of gravity of the respective sample, to name but a few of the possible examples.

The initial sample fingerprint data may comprise an initial chromatogram of the respective sample. Specifically, the initial chromatogram may comprise a continuous chromatogram or comprise a segmented chromatogram. The current sample fingerprint data may comprise a current chromatogram of the specific sample. Specifically, the current chromatogram may comprise a continuous chromatogram or comprise a segmented chromatogram.

Mapping the current sample fingerprint data to the initial sample fingerprint data may comprise mapping the current chromatogram to the initial chromatogram of one of the plurality of samples in order to identify the specific sample. Such mapping may, for example, be implemented by one or more of a matching algorithm, a comparison algorithm, and/or a trained machine learning model. Thereby, a particularly efficient means for sample tracking and/or identification may be achieved.

A chromatogram may be understood herein as results obtained via chromatography, such as for example via liquid chromatography. For example, chromatography may be a technique used to separate and analyze the components of a respective sample. In a chromatogram, the x-axis may represent time or distance traveled by the components, while the y-axis may represent a signal intensity or concentration of the separated components. Each component may appear in a respective chromatogram as a peak or band in the chromatogram, with its position, shape, and size providing information about its identity and quantity in the respective sample.

By providing the above discussed initial and/or current sample fingerprint data, a particularly efficient means for sample tracking and/or identification may be achieved. Specifically, it becomes possible to obtain the initial and/or current sample fingerprint data without needing to access the sample (e.g., by opening the sample vessel and/or drawing from the sample) and/or with high precision.

In the above, several potential examples for the initial and current sample fingerprint data have been given and discussed. It is understood, however, that the present disclosure is not limited to these specific examples, and other types of initial and current sample fingerprint data may be used. Furthermore, the initial and current sample fingerprint data may comprise two or more different types of fingerprint data, such as infrared vibrational spectral data (e.g., optical) and conductivity and/or impedance physical parameters (e.g., electrical/electrochemical).

A machine learning algorithm may be configured to perform the step of mapping the current sample fingerprint data to the initial sample fingerprint data. In particular, the current sample fingerprint data may be provided to the machine learning algorithm, wherein the machine learning algorithm may be configured to access at least the registered initial sample fingerprint data. The machine learning algorithm may be a trained machine learning model. The trained machine learning model may be trained using one of plurality of known techniques, such as via a random forest technique.

The machine learning algorithm and/or the trained machine learning model may be trained using historic registered initial sample fingerprint data and historic registered current sample fingerprint data.

The trained machine learning model may be trained using a training set. The training set may comprise a first training set comprising a plurality of first training elements. Each first training element may respectively comprise historic registered initial sample fingerprint data and historic current sample fingerprint data previously mapped thereto. In particular, it may therefore be possible to train the trained machine learning model using the data historically obtained by execution of the disclosed method itself. Furthermore, each time the trained machine learning model maps current sample fingerprint data to respective initial sample fingerprint data, a corresponding new entry into the first training set may be made. Thereby, the first training set may expand the longer the current method is implemented.

The training set may comprise a second training set comprising a plurality of second training elements. Each second training element may be obtained via a respective training sample. Specifically, a plurality of training samples may be provided and initial sample fingerprint data of each of the plurality of training samples may be registered and/or determined. Furthermore, for each training sample, current sample fingerprint data may be obtained. Each second training element may comprise the initial sample fingerprint data obtained from a respective training sample and the current sample fingerprint data obtained from the respective training sample.

Furthermore, the trained machine learning model may be re-trained using the training set and/or the first training set once a retraining condition is met. The retraining condition may be a predetermined time interval measured from the time of the last training of the trained machine learning model and/or the retraining condition may be a predetermined numerical threshold corresponding to a number of new entries into the first training set since the last training of the trained machine learning model.

A further aspect of the present invention relates to a sample processing system configured to process samples. The sample processing system may be configured to process at least one sample, preferably a plurality of samples.

In other words, the sample processing system may be configured to process one or more samples. In particular, the sample processing system may be configured to transport the one or more sample and/or to analyse the one or more sample. In particular, the sample processing system may comprise a sample transport unit configured to transport the one or more samples at least within the sample processing system. The sample processing system may comprise a sample analysis unit configured to analyse the one or more samples. However, it is understood that the sample processing system is not limited thereto, and thus the sample processing system may be configured to perform alternative and/or additional processing steps. For example, the sample processing system may be configured to perform one or more of preparing the one or more samples, treating the one or more samples, pre-treating the one or more samples, storing the one or more samples, heating the one or more samples, freezing the one or more samples.

The sample processing system may, for example, be an in-vitro diagnostic analyzer. However, other types of sample processing systems may also be used, such as other types of analytical systems and/or analytical devices.

The sample processing system comprises a registration unit. The registration unit is configured to configured to register, for each sample of a plurality of samples to be processed by the sample processing system, initial sample identification data uniquely identifying the respective sample among the plurality of samples.

The registration unit is further configured to register, for each sample of the plurality of samples, initial sample fingerprint data indicative of an internal state of the respective sample. The registration unit may comprise one or more sensors and/or measurement units configured to measure and/or determine, for each sample of the plurality of samples, the initial sample fingerprint data and/or the initial sample identification data.

The registration unit is further configured to associate, for each sample of the plurality of samples, the respective initial sample identification data with the respective initial sample fingerprint data.

While the registration unit has been described above as being configured to register both initial sample identification data and initial sample fingerprint data, as well as to associate said respective initial sample identification data with the respective initial sample fingerprint data, it is understood that the registration unit is not limited thereto. Thus, while the registration unit may be implemented as a single registration unit, the registration unit may alternatively comprise two or more registration sub-units. Each registration sub-unit may be configured to perform one or more of: register, for each sample of a plurality of samples to be processed by the sample processing system, initial sample identification data uniquely identifying the respective sample among the plurality of samples; register, for each sample of the plurality of samples, initial sample fingerprint data indicative of an internal state of the respective sample; and associate, for each sample of the plurality of samples, the respective initial sample identification data with the respective initial sample fingerprint data. For example, the registration unit may comprise a first registration sub-unit configured to register the initial sample identification data, a second registration sub-unit configured to register the initial sample fingerprint data, and a third registration sub-unit configured to associate the respective initial sample identification data with the respective initial sample fingerprint data.

The sample processing system further comprises an identification triggering unit configured to determine that a sample identification condition is met, wherein the sample identification condition is indicative of a need to identify a specific sample being processed by the sample processing system.

The sample processing system further comprises a sample measurement unit configured to measure, for the specific sample, current sample fingerprint data indicative of a current internal state of the specific sample.

While the sample measurement unit is described as a separate unit herein, it is understood that the registration unit and/or the second registration sub-unit may also be configured as and/or comprise the sample measurement unit. Thus, the registration unit may be configured to register the initial sample fingerprint data and may be further configured as the sample measurement unit, i.e., configured to measure, for the specific sample, current sample fingerprint data indicative of a current internal state of the specific sample. Alternatively, the sample measurement unit may be configured as a registration sub-unit and/or the registration unit may comprise the sample measurement unit.

The sample processing system further comprises a sample identification unit configured to map the current sample fingerprint data to the initial sample fingerprint data of one sample of the plurality of samples in order to identify the specific sample.

The sample processing system may in particular comprise one or more features of the method, as described herein.

The sample processing system may further comprise an evaluation unit configured to evaluate the initial sample fingerprint data and/or the current sample fingerprint data and group the plurality of samples into one or more sample groups, wherein each group may be indicative of correspondingly grouped samples being drawn from the same individual. As already discussed for the method above, the evaluation unit may comprise a trained grouping machine learning model.

The sample processing system may comprise one or more sensors, such as optical, electrical, electronic, haptic, and/or weight sensors, configured to determine that the sample identification condition is met.

The registration unit may further comprise an input element, e.g., a first reception element, configured to receive the initial sample identification data as input, such as for example by direct operator input and/or from a database. Alternatively, the input element may be configured to measure and/or determine the initial sample identification data prior and/or concurrent to its registration.

The registration unit may further comprise a second reception element. The input element and/or the second reception element may be configured to receive the initial sample fingerprint data as input, such as for example by direct operator input and/or from a database. Alternatively, the input element and/or the second reception element may be configured to measure and/or determine the initial sample fingerprint data prior and/or concurrent to its registration.

The sample processing system may further comprise one or more databases configured to store at least one of the initial sample identification data, the initial sample fingerprint data, and/or the current sample fingerprint data.

The registration unit and/or the second registration sub-unit and/or the sample measurement unit may comprise an infrared vibrational spectroscopy unit. The infrared vibrational spectroscopy unit of the registration unit and/or of the sample measurement unit may be configured to measure infrared vibrational spectral data, such as the initial infrared vibrational spectral data and/or current infrared vibrational spectral data, of one or more samples of the plurality of samples. The sample identification unit may be configured to map the current infrared vibrational spectral data to the initial infrared vibrational spectral data of one of the plurality of samples in order to identify the specific sample.

The registration unit and/or the second registration sub-unit and/or the sample measurement unit may comprise a microwave spectroscopy unit (e.g., a microwave rotational spectroscopy unit). The microwave spectroscopy unit of the registration unit and/or of the sample measurement unit may be configured to measure microwave spectral data, such as initial microwave spectral data and/or current microwave spectral data, of one or more samples of the plurality of samples. The sample identification unit may be configured to map the current microwave spectral data to the initial microwave spectral data of one of the plurality of samples in order to identify the specific sample.

The registration unit and/or the second registration sub-unit and/or the sample measurement unit may comprise a Raman spectroscopy unit. The Raman spectroscopy unit of the registration unit and/or of the sample measurement unit may be configured to measure Raman spectral data, such as initial Raman spectral data and/or current Raman spectral data, of one or more samples of the plurality of samples. The sample identification unit may be configured to map the current Raman spectral data to the initial Raman spectral data of one of the plurality of samples in order to identify the specific sample.

The registration unit and/or the second registration sub-unit and/or the sample measurement unit may comprise a NMR spectroscopy unit. The NMR spectroscopy unit of the registration unit and/or of the sample measurement unit may be configured to measure NMR spectral data, such as initial NMR spectral data and/or current NMR spectral data, of one or more samples of the plurality of samples. The sample identification unit may be configured to map the current NMR spectral data to the initial NMR spectral data of one of the plurality of samples in order to identify the specific sample.

The registration unit and/or the second registration sub-unit and/or the sample measurement unit may comprise an absorption (e.g., ultraviolet-visible) spectroscopy unit. The absorption (e.g., ultraviolet-visible) spectroscopy unit of the registration unit and/or of the sample measurement unit may be configured to measure absorption (e.g., ultraviolet-visible) spectral data, such as initial absorption (e.g., ultraviolet-visible) spectral data and/or current absorption (e.g., ultraviolet-visible) spectral data, of one or more samples of the plurality of samples. The sample identification unit may be configured to map the current absorption (e.g., ultraviolet-visible) spectral data to the initial absorption (e.g., ultraviolet-visible) spectral data of one of the plurality of samples in order to identify the specific sample.

The registration unit and/or the second registration sub-unit and/or the sample measurement unit may comprise a mass spectrometry unit. The mass spectrometry unit of the registration unit and/or of the sample measurement unit may be configured to measure mass spectrometry spectral data, such as initial mass spectrometry spectral data and/or current mass spectrometry spectral data, of one or more samples of the plurality of samples. The sample identification unit may be configured to map the current mass spectrometry spectral data to the initial mass spectrometry spectral data of one of the plurality of samples in order to identify the specific sample.

The registration unit and/or the second registration sub-unit and/or the sample measurement unit may comprise physical parameter measurement unit. The physical parameter measurement unit of the registration unit and/or of the sample measurement unit may be configured to measure values of a plurality of physical parameters, such as initial values of a plurality of physical parameters and/or current values of a plurality of physical parameters, of one or more samples of the plurality of samples. The sample identification unit may be configured to map the current values of a plurality of physical parameters to the initial values of a plurality of physical parameters of one of the plurality of samples in order to identify the specific sample.

The registration unit and/or the second registration sub-unit and/or the sample measurement unit may comprise a chromatography unit. The chromatography unit of the registration unit and/or of the sample measurement unit may be configured to measure chromatogram data, such as initial chromatogram data and/or current chromatogram data, of one or more samples of the plurality of samples. The sample identification unit may be configured to map the current chromatogram data to the initial chromatogram data of one of the plurality of samples in order to identify the specific sample.

The sample identification unit may comprise a machine learning unit. The machine learning unit may be configured to map the current sample fingerprint data to the initial sample fingerprint data of one sample of the plurality of samples in order to identify the specific sample. The machine learning unit may comprise a trained machine learning model configured to receive, as input, the current sample fingerprint data. The trained machine learning model may be configured to map the received current sample fingerprint data to the initial sample fingerprint data of one sample of the plurality of samples in order to identify the specific sample. The trained machine learning model may be trained using one of plurality of known techniques, as discussed for the method above.

A further aspect of the present disclosure concerns a non-transitory computer-readable storage medium containing instructions that, when executed by a computing unit, cause the computing unit to perform the method for sample identification, as described herein. In particular, the non-transitory storage medium may comprise one or more features, as described herein for the method for sample identification and/or the sample processing system.

In the following, aspects, features, and embodiments of the present disclosure are further explained and/or illustrated using the appended, exemplary Figures. It is understood that said Figures are of illustrative nature, and the present disclosure is therefore not limited to the shown embodiments of the Figures. The Figures show:
**FIG. 1** illustrates a flow chart of an exemplary method according to the present disclosure.
**FIG. 2** illustrates a schematic overview over an exemplary sample processing system configured to process samples according to the present disclosure.
**FIG. 3** illustrates a schematic overview over a further exemplary sample processing system configured to process samples according to the present disclosure.
**FIG. 4** illustrates a schematic overview over an exemplary registration unit and exemplary second registration sub-unit according to the present disclosure.
**FIG. 5** illustrates a schematic overview over an exemplary sample measurement unit according to the present disclosure.

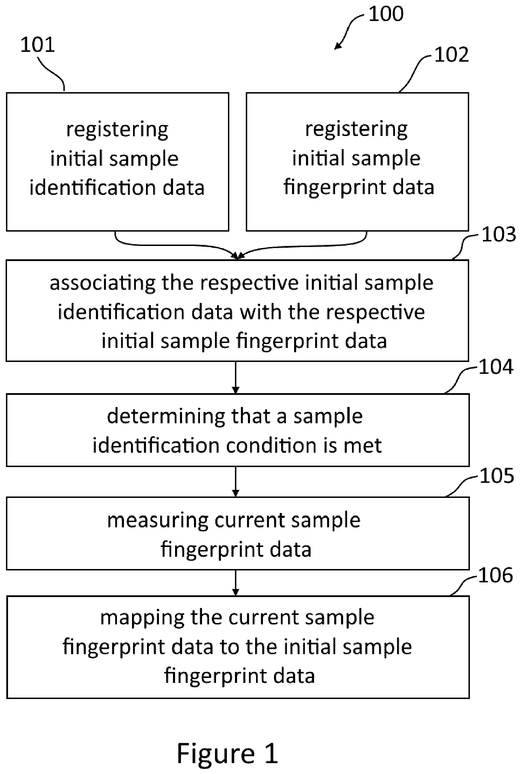

**Figure 1** shows a flow chart of an exemplary method **100** according to the present disclosure. In particular, the method **100** is a method **100** of sample identification of a sample in a sample processing system. It is to be understood that in particular the sequence of described method steps is illustrative only, and that other sequences of the method steps may be possible.

In an exemplary first step **101,** initial sample identification data may be registered for each sample of a plurality of samples to be processed by the sample processing system. The initial sample identification data of a particular sample may uniquely identify the particular sample among the plurality of samples. For example, the initial sample identification data may comprise a (e.g., unique) sample ID for the respective sample. The initial sample identification data may be stored in a common database.

In an exemplary second step **102,** initial sample fingerprint data may be registered for each sample of the plurality of samples to be processed by the sample processing system. The initial sample fingerprint data may be indicative of an internal state, e.g., an internal chemical state, of the respective sample. For example, the initial sample fingerprint data may comprise an infrared vibrational spectrum of the respective sample. The initial sample fingerprint data may be stored in the common database.

It is noted that step **101** and step **102** may be performed at least partially or fully simultaneously. However, the present disclosure is not limited thereto. Therefore, for example, step **101** may be performed at least partially prior to step **102,** or step **102** may be performed at least partially prior to step **101.** In other words, a sequence of steps **101** and **102** may be chosen according to particular needs and/or requirements of the user and/or operator.

In an exemplary third step **103,** respective initial sample identification data and respective initial sample fingerprint data may be associated for each sample of the plurality of samples. For example, for each sample, an association data element may be generated to store and/or register the respective association between respective initial sample identification data and respective initial sample fingerprint data. For example, an association data element may be a row or column of a database table containing at least the respective initial sample identification data and respective initial sample fingerprint data. The association(s) and/or the association data element(s) may be stored in the common database.

In an exemplary fourth step **104,** it may be determined that a sample identification condition is met. The sample identification condition may be indicative of a need to identify a specific sample being processed by the sample processing system. For example, it may be determined, e.g., via a camera and/or optical sensor, that a sample label of a particular sample of the plurality of samples is not readable (e.g., the label is determined to be unreadable). Consequently, it may be determined that therefore an unreadable- and/or missing-label condition indicative of a label of the respective sample not being readable or missing from the sample is met.

In an exemplary fifth step **105,** current sample fingerprint data indicative of a current internal state of the specific sample may be measured for the specific sample. For example, the step of measuring may comprise measuring a current infrared vibrational spectrum of the specific sample, wherein the current sample fingerprint data may comprise the current infrared vibrational spectrum of the respective sample. Merely as an example, the current sample fingerprint data may also be stored in the common database.

In an exemplary sixth step **106,** the current sample fingerprint data may be mapped and/or matched to the initial sample fingerprint data of one sample of the plurality of samples. In particular, it may therefore be possible to identify the specific sample as the one sample of the plurality of samples. Furthermore, the current sample fingerprint data may also be associated with the initial sample fingerprint data of the one sample of the plurality of samples.

In particular, steps **105** and **106** may be performed in response to the determination of the sample identification condition being met in step **104.** In other words, performing steps **105** and **106** may be conditional on the determination of the sample identification condition being met in step **104.**

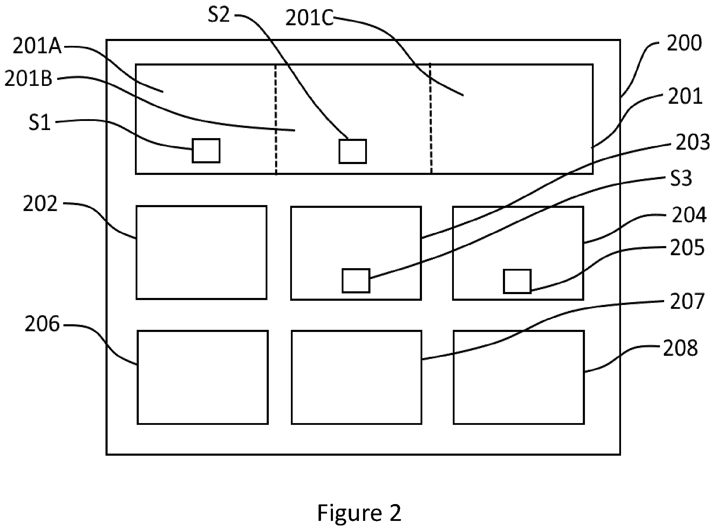

**Figure 2** shows a schematic overview of an exemplary sample processing system **200** configured to process samples. Specifically, the sample processing system **200** may be configured to process at least one sample, preferably a plurality of samples. In the shown exemplary embodiment, the sample processing system **200** may be an in-vitro diagnostic analyzer.

The exemplary sample processing system **200** comprises a registration unit **201.** The registration unit **201** may be configured to register both initial sample identification data and initial sample fingerprint data, as well as to associate said respective initial sample identification data with the respective initial sample fingerprint data. While the registration unit **201** may be implemented as a single registration unit (indicated by the dashed lines between registration sub-units **201A, 201B**, **201C),** the shown exemplary registration unit **201** may comprise three registration sub-units **201A, 201B**, **201C.**

The registration unit **201** and/or the first registration sub-unit **201A** may be configured to register, for each sample of a plurality of samples to be processed by the sample processing system **200,** initial sample identification data uniquely identifying the respective sample among the plurality of samples. Furthermore, the registration unit **201** and/or the first registration sub-unit **201A** may comprise one or more first sensors **S1** configured to measure and/or determine the initial sample identification data. The first sensors **S1** may, merely as an example, comprise one or more optical sensor(s), haptic sensor(s), electrical sensor(s), acoustic sensor(s), and/or the like.

The registration unit **201** and/or the second registration sub-unit **201B** may be further configured to register, for each sample of the plurality of samples, initial sample fingerprint data indicative of an internal state of the respective sample. Furthermore, the registration unit **201** and/or the second registration sub-unit **201B** may comprise one or more second sensors **S2** configured to measure and/or determine the initial sample fingerprint data. The second sensors **S2** may, merely as an example, comprise one or more optical sensor(s), haptic sensor(s), electrical sensor(s), acoustic sensor(s), and/or the like.

The registration unit **201** and/or the third registration sub-unit **201C** may further be configured to associate, for each sample of the plurality of samples, the respective initial sample identification data with the respective initial sample fingerprint data.

The sample processing system **200** may further comprise an identification triggering unit **202** configured to determine that a sample identification condition is met, wherein the sample identification condition is indicative of a need to identify a specific sample being processed by the sample processing system **200.**

The sample processing system **200** may further comprise a sample measurement unit **203** configured to measure, for the specific sample, current sample fingerprint data indicative of a current internal state of the specific sample. In the shown exemplary sample processing system **200** of Figure 2, the sample measurement unit **203** is provided as separate to the registration unit **201** and/or the second registration sub-unit **201B.** Furthermore, the sample measurement unit **203** may comprise one or more third sensors **S3** configured to measure and/or determine the current sample fingerprint data. The third sensors **S3** may, merely as an example, comprise one or more optical sensor(s), haptic sensor(s), electrical sensor(s), acoustic sensor(s), and/or the like.

The exemplary sample processing system **200** may further comprise a sample identification unit **204** configured to map the current sample fingerprint data to the initial sample fingerprint data of one sample of the plurality of samples in order to identify the specific sample. In particular, the sample identification unit **204** may be configured to therefore identify the specific sample among the plurality of samples. The sample identification unit **204** may for example be implemented as and/or comprise a machine learning unit **205.** The machine learning unit **205** may be configured to map the current sample fingerprint data to the initial sample fingerprint data of one sample of the plurality of samples in order to identify the specific sample, as discussed above.

The sample processing system **200** may be configured to transport the one or more sample and/or to analyse the one or more sample. Specifically, the exemplary sample processing system **200** may comprise a sample transport unit **206** configured to transport the one or more samples at least within the sample processing system **200.** The sample transport unit **206** may be implemented in a conventional manner, and may for example comprise one or more conveyance elements, belts, grippers, robotic elements, and the like to move the sample within the sample processing system **200.**

The exemplary sample processing system **200** may further comprise an evaluation unit **207** configured to evaluate the initial sample fingerprint data and/or the current sample fingerprint data and group the plurality of samples into one or more sample groups, wherein each group may be indicative of correspondingly grouped samples being drawn from the same individual.

The exemplary sample processing system **200** may further comprise a sample analysis unit **208** configured to analyse the one or more samples. The sample analysis unit **208** may be implemented in a conventional manner and comprise one or more analysis elements configured to analyse the one or more samples. Specifically, the choice of which analysis elements to provide may be dependent on the desired operational capabilities of the sample processing system **200.** Merely as an example, a sample processing system **200** configured to perform at least mass spectrometry analysis may comprise at least a mass spectrometry analysis element. However, the sample analysis unit **208** may be configured to provide further functionality, and thus comprise one or more functional processing elements. The one or more functional processing elements may be configured to, for example, perform one or more of preparing the one or more samples, treating the one or more samples, pre-treating the one or more samples, storing the one or more samples, heating the one or more samples, freezing the one or more samples.

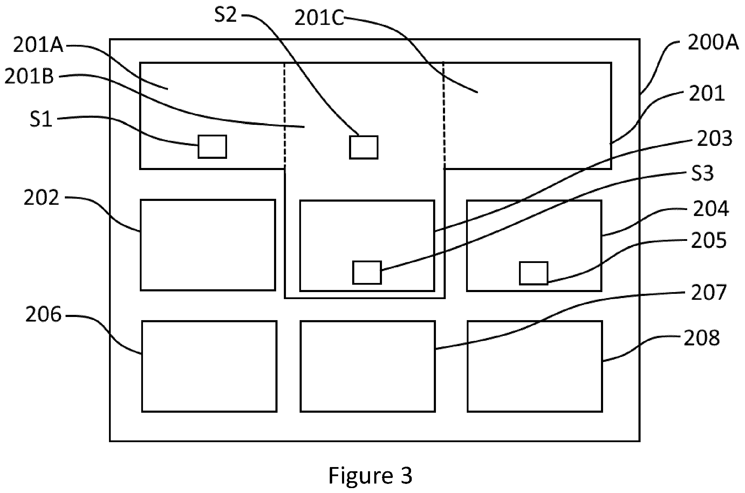

**Figure 3** shows a schematic overview of a further exemplary sample processing system **200A** configured to process samples. Specifically, the sample processing system **200A** of Figure 3 is substantially similar to the sample processing system 200 shown in Figure 2, such that in the following only the differences therebetween will be discussed.

Specifically, in contrast to the embodiment shown in Figure 2, the exemplary sample processing system **200A** may be configured such that the registration unit **201** and/or the second registration sub-unit **201B** may also be configured as and/or comprise the sample measurement unit **203.**

Consequently, the registration unit **201,** and/or the second registration sub-unit **201B,** and/or the sample measurement unit **203** may be configured to measure, for each sample of the plurality of samples, initial sample fingerprint data indicative of an internal state of the respective sample, and to further measure, for the specific sample, current sample fingerprint data indicative of a current internal state of the specific sample.

It is noted that, while Figure 3 shows both the provision of second sensors **S2** and third sensors **S3,** the present disclosure is not limited thereto. In particular, for the shown exemplary sample processing system **200A,** only third sensors **S3** may be provided.

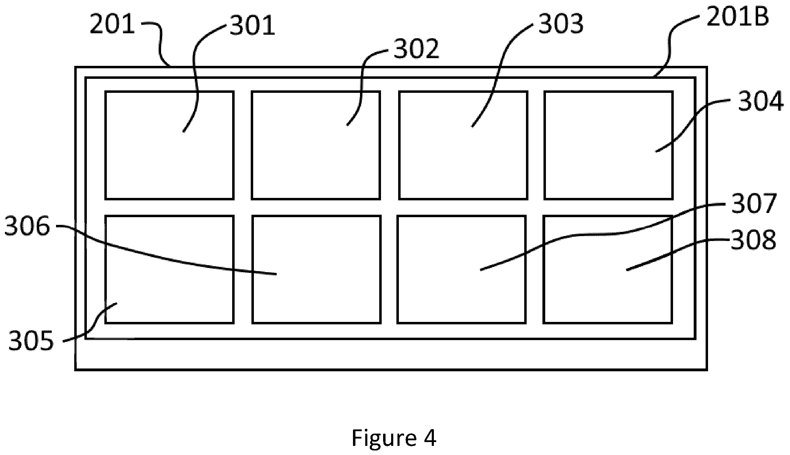

**Figure 4** shows a schematic overview over an exemplary registration unit **201** and exemplary second registration sub-unit **201B.** Potential provision of a first registration sub-unit and/or a third registration sub-unit has been omitted for illustrative reasons in Figure 4.

The registration unit **201** and/or the second registration sub-unit **201B** may comprise a first infrared vibrational spectroscopy unit **301.** The first infrared vibrational spectroscopy unit **301** may be configured to measure infrared vibrational spectral data, such as the initial infrared vibrational spectral data and/or current infrared vibrational spectral data, of one or more samples of the plurality of samples.

The registration unit **201** and/or the second registration sub-unit **201B** may comprise a first Raman spectroscopy unit **302.** The first Raman spectroscopy unit **302** may be configured to measure Raman spectral data, such as initial Raman spectral data and/or current Raman spectral data, of one or more samples of the plurality of samples.

The registration unit **201** and/or the second registration sub-unit **201B** may comprise a first NMR spectroscopy unit **303.** The first NMR spectroscopy unit **303** may be configured to measure NMR spectral data, such as initial NMR spectral data and/or current NMR spectral data, of one or more samples of the plurality of samples.

The registration unit **201** and/or the second registration sub-unit **201B** may comprise a first absorption (e.g., ultraviolet-visible) spectroscopy unit **304.** The first absorption (e.g., ultraviolet-visible) spectroscopy unit **304** may be configured to measure absorption (e.g., ultraviolet-visible) spectral data, such as initial absorption (e.g., ultraviolet-visible) spectral data and/or current absorption (e.g., ultraviolet-visible) spectral data, of one or more samples of the plurality of samples.

The registration unit **201** and/or the second registration sub-unit **201B** may comprise a first mass spectrometry unit **305.** The first mass spectrometry unit **305** may be configured to measure mass spectrometry spectral data, such as initial mass spectrometry spectral data and/or current mass spectrometry spectral data, of one or more samples of the plurality of samples.

The registration unit **201** and/or the second registration sub-unit **201B** may comprise a first physical parameter measurement unit **306.** The first physical parameter measurement unit **306** may be configured to measure values of a plurality of physical parameters, such as initial values of a plurality of physical parameters and/or current values of a plurality of physical parameters, of one or more samples of the plurality of samples.

The registration unit **201** and/or the second registration sub-unit **201B** may comprise a first chromatography unit **307.** The first chromatography unit 307 may be configured to measure chromatogram data, such as initial chromatogram data and/or current chromatogram data, of one or more samples of the plurality of samples.

The registration unit **201** and/or the second registration sub-unit **201B** may comprise a first microwave spectroscopy unit **308.** The first microwave spectroscopy unit **308** may be configured to measure microwave spectral data, such as initial microwave spectral data and/or current microwave spectral data, of one or more samples of the plurality of samples.

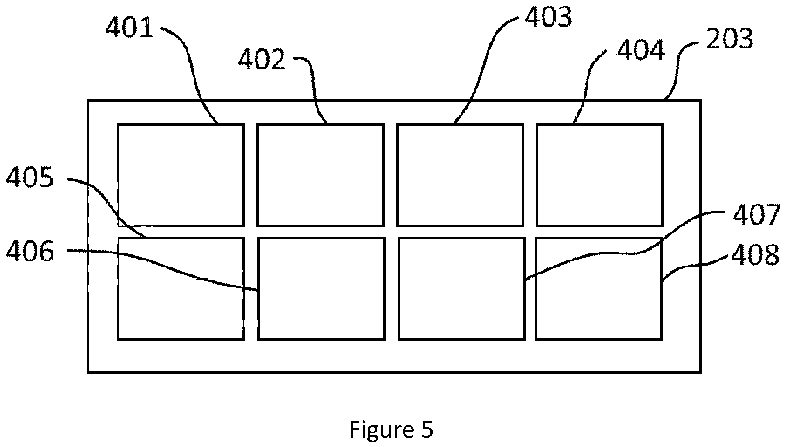

**Figure 5** shows a schematic overview over an exemplary sample measurement unit **203.**

The sample measurement unit **203** may comprise a second infrared vibrational spectroscopy unit **401.** The second infrared vibrational spectroscopy unit **401** may be configured to measure infrared vibrational spectral data, such as the initial infrared vibrational spectral data and/or current infrared vibrational spectral data, of one or more samples of the plurality of samples.

The sample measurement unit **203** may comprise a second Raman spectroscopy unit **402.** The second Raman spectroscopy unit **402** may be configured to measure Raman spectral data, such as initial Raman spectral data and/or current Raman spectral data, of one or more samples of the plurality of samples.

The sample measurement unit **203** may comprise a second NMR spectroscopy unit **403.** The second NMR spectroscopy unit **403** may be configured to measure NMR spectral data, such as initial NMR spectral data and/or current NMR spectral data, of one or more samples of the plurality of samples.

The sample measurement unit **203** may comprise a second absorption (e.g., ultraviolet-visible) spectroscopy unit **404.** The second absorption (e.g., ultraviolet-visible) spectroscopy unit **404** may be configured to measure absorption (e.g., ultraviolet-visible) spectral data, such as initial absorption (e.g., ultraviolet-visible) spectral data and/or current absorption (e.g., ultraviolet-visible) spectral data, of one or more samples of the plurality of samples.

The sample measurement unit **203** may comprise a second mass spectrometry unit **405.** The second mass spectrometry unit **405** may be configured to measure mass spectrometry spectral data, such as initial mass spectrometry spectral data and/or current mass spectrometry spectral data, of one or more samples of the plurality of samples.

The sample measurement unit **203** may comprise a second physical parameter measurement unit **406.** The second physical parameter measurement unit **406** may be configured to measure values of a plurality of physical parameters, such as initial values of a plurality of physical parameters and/or current values of a plurality of physical parameters, of one or more samples of the plurality of samples.

The sample measurement unit **203** may comprise a second chromatography unit **407.** The second chromatography unit **407** may be configured to measure chromatogram data, such as initial chromatogram data and/or current chromatogram data, of one or more samples of the plurality of samples.

The sample measurement unit **203** may comprise a second microwave spectroscopy unit **408.** The second microwave spectroscopy unit **408** may be configured to measure microwave spectral data, such as initial microwave spectral data and/or current microwave spectral data, of one or more samples of the plurality of samples.

It is noted that the present invention is not to be interpreted as being limited to any of the particular, exemplary embodiments shown in the Figures and/or described herein.

**REFERENCE SIGNS**

| | |
|---|---|
| **100** | method |
| **101, 102, 103, 104, 105, 106** | steps |
| **200, 200A** | sample processing system |
| **201** | registration unit |
| **201A** | first registration sub-unit |
| **201B** | second registration sub-unit |
| **201C** | third registration sub-unit |
| **202** | identification triggering unit |
| **203** | sample measurement unit |
| **204** | sample identification unit |
| **205** | machine learning unit |
| **206** | sample transport unit |
| **207** | evaluation unit |
| **208** | sample analysis unit |
| **S1, S2, S3** | sensor(s) |
| **301, 401** | infrared vibrational spectroscopy unit |
| **302, 402** | Raman spectroscopy unit |
| **303, 403** | NMR spectroscopy unit |
| **304, 404** | absorption spectroscopy unit |
| **305, 405** | mass spectrometry unit |
| **306, 406** | physical parameter measurement unit |
| **307, 407** | chromatography unit |
| **308, 408** | microwave spectroscopy unit |

## Claims

1. Method (100) of sample identification of a sample in a sample processing system (200, 200A), comprising:
registering, for each sample of a plurality of samples to be processed by the sample processing system (200, 200A), initial sample identification data uniquely identifying the respective sample among the plurality of samples;
registering, for each sample of the plurality of samples, initial sample fingerprint data indicative of an internal state of the respective sample;
associating, for each sample of the plurality of samples, the respective initial sample identification data with the respective initial sample fingerprint data;
determining that a sample identification condition is met, wherein the sample identification condition is indicative of a need to identify a specific sample being processed by the sample processing system (200, 200A);
measuring, for the specific sample, current sample fingerprint data indicative of a current internal state of the specific sample; and
mapping the current sample fingerprint data to the initial sample fingerprint data of one sample of the plurality of samples in order to identify the specific sample.

2. The method (100) according to claim 1, wherein the initial sample identification data comprises one or more of:
a sample ID,
sample origin data, and
a sample processing plan.

3. The method (100) according to claim 1 or 2, wherein registering the initial sample fingerprint data comprises receiving the initial sample fingerprint data as input; or
wherein registering the initial sample fingerprint data comprises measuring and/or determining the initial sample fingerprint data of the respective sample.

4. The method (100) according to any one of claims 1 to 3, wherein each sample of the plurality of samples is one or more of a blood serum sample, a blood plasma sample, a saliva sample, and a urine sample.

5. The method (100) according to any one of claims 1 to 4, wherein each sample of the plurality of samples is one of a solid sample, a liquid sample, or a gaseous sample.

6. The method (100) according to any one of claims 1 to 5, wherein the initial sample fingerprint data comprises initial infrared vibrational spectral data of the respective sample,
wherein the current sample fingerprint data comprises current infrared vibrational spectral data of the specific sample, and
wherein mapping the current sample fingerprint data to the initial sample fingerprint data comprises mapping the current infrared vibrational spectral data to the initial infrared vibrational spectral data of one of the plurality of samples in order to identify the specific sample.

7. The method (100) according to any one of claims 1 to 6, wherein the initial sample fingerprint data comprises initial Raman spectral data of the respective sample, wherein the current sample fingerprint data comprises current Raman spectral data of the specific sample, and wherein mapping the current sample fingerprint data to the initial sample fingerprint data comprises mapping the current Raman spectral data to the initial Raman spectral data of one of the plurality of samples in order to identify the specific sample; and/or
wherein the initial sample fingerprint data comprises initial microwave spectral data of the respective sample, wherein the current sample fingerprint data comprises current microwave spectral data of the specific sample, and wherein mapping the current sample fingerprint data to the initial sample fingerprint data comprises mapping the current microwave spectral data to the initial microwave spectral data of one of the plurality of samples in order to identify the specific sample.

8. The method (100) according to any one of claims 1 to 7, wherein the initial sample fingerprint data comprises initial NMR spectral data of the respective sample,
wherein the current sample fingerprint data comprises current NMR spectral data of the specific sample, and
wherein mapping the current sample fingerprint data to the initial sample fingerprint data comprises mapping the current NMR spectral data to the initial NMR spectral data of one of the plurality of samples in order to identify the specific sample.

9. The method (100) according to any one of claims 1 to 8, wherein the initial sample fingerprint data comprises an initial absorption spectrum of the respective sample,
wherein the current sample fingerprint data comprises a current absorption spectrum of the specific sample, and
wherein mapping the current sample fingerprint data to the initial sample fingerprint data comprises mapping the current absorption spectrum to the initial absorption spectrum of one of the plurality of samples in order to identify the specific sample.

10. The method (100) according to any one of claims 1 to 9, wherein the initial sample fingerprint data comprises an initial mass spectrometry spectrum of the respective sample,
wherein the current sample fingerprint data comprises a current mass spectrometry spectrum of the specific sample, and
wherein mapping the current sample fingerprint data to the initial sample fingerprint data comprises mapping the current mass spectrometry spectrum to the initial mass spectrometry spectrum of one of the plurality of samples in order to identify the specific sample.

11. The method (100) according to any one of claims 1 to 10, wherein the initial sample fingerprint data comprises initial values of a plurality of physical parameters of the respective sample,
wherein the current sample fingerprint data comprises current values of the plurality of physical parameters of the specific sample, and
wherein mapping the current sample fingerprint data to the initial sample fingerprint data comprises mapping the current values to the initial values of one of the plurality of samples in order to identify the specific sample.

12. The method (100) according to any one of claims 1 to 11, wherein the initial sample fingerprint data comprises an initial chromatogram of the respective sample,
wherein the current sample fingerprint data comprises a current chromatogram of the specific sample, and
wherein mapping the current sample fingerprint data to the initial sample fingerprint data comprises mapping the current chromatogram to the initial chromatogram of one of the plurality of samples in order to identify the specific sample.

13. The method (100) according to any one of claims 1 to 12, wherein a machine learning algorithm is configured to perform the step of mapping the current sample fingerprint data to the initial sample fingerprint data, and, optionally,
wherein the machine learning algorithm is trained using historic registered initial sample fingerprint data and historic registered current sample fingerprint data.

14. A sample processing system (200, 200A) configured to process samples, comprising:
a registration unit (201) configured to register, for each sample of a plurality of samples to be processed by the sample processing system (200, 200A), initial sample identification data uniquely identifying the respective sample among the plurality of samples,
wherein the registration unit (201) is further configured to register, for each sample of the plurality of samples, initial sample fingerprint data indicative of an internal state of the respective sample, and is further configured to associate, for each sample of the plurality of samples, the respective initial sample identification data with the respective initial sample fingerprint data;
an identification triggering unit (202) configured to determine that a sample identification condition is met, wherein the sample identification condition is indicative of a need to identify a specific sample being processed by the sample processing system (200, 200A);
a sample measurement unit (203) configured to measure, for the specific sample, current sample fingerprint data indicative of a current internal state of the specific sample; and
a sample identification unit (204) configured to map the current sample fingerprint data to the initial sample fingerprint data of one sample of the plurality of samples in order to identify the specific sample.

15. A non-transitory computer-readable storage medium containing instructions that, when executed by a computing unit, cause the computing unit to perform the method (100) according any one of claims 1 to 13.
